# EUROPEAN PATENT APPLICATION

(11) **EP 1 883 107 A2**
(43) Date of publication of application: **30.01.2008**
(21) Application number: 07013364.0
(22) Date of filing: 09.07.2007
(51) Int. Cl.: H01L 23/31, A61N 1/375

(54) **Method for forming packaged microelectronic devices and devices thus obtained**

(30) Priority: 07.07.2006 US 819076 P
(71) Applicant: Interuniversitair Microelektronica Centrum, 3001 Leuven (BE)
(72) Inventor: Vanden Bulcke, Mathieu, 5030 Gembloux (BE); Beyne, Eric, 3001 Leuven (BE)
(74) Representative: Bird, William Edward

(57) **Abstract**

The present invention provides a packaged microelectronic device (20) comprising at least one electrode (10) comprising a chip (18) embedded in a package. The chip (18) comprises a back electrode (17) located at a first side of the chip (18), and electronic circuitry (14) located at a second side of the chip (18), the second side being opposite to the first side, and wherein the back electrode (17) is part of the package. The present invention furthermore provides a method for forming such packaged microelectronic devices (20).

## Description

### Technical field of the invention

The present invention relates to the field of microelectronic packaging technology. More particularly, the present invention relates to a packaged microelectronic device and to a method for the formation of such a packaged microelectronic device. The packaged microelectronic device according to embodiments of the present invention may be used as medical or biomedical implants.

### Background of the invention

Microelectronic devices are nowadays widely used in all kinds of technologies, for example in medical or biomedical implants. In the area of medical implants, cochlear prostheses are one of the main driving applications for new technological developments. Although such devices have been obtained worldwide, there still is a need for devices with improved performances, e.g. increased number of channels or electrodes (see "The past, present, and future of cochlear prostheses," F. Spelman in Engineering in Medicine and Biology Magazine, IEEE , vol. 18, no.3, pp. 27-33, May/Jun 1999 and "Cochlear implants. Fundamentals and applications", by Graeme Clark, Springer-Verlag, New York, 2003). And it is not only in the field of cochlear prostheses, of more in general medical implants, that there is a need for devices with a high number of channels or electrodes. Generally speaking, in the field of electro-stimulated biomedical implants, higher electrode densities are becoming of growing interest.

At the same time, the importance of MEMS (Micro-Electro-Mechanical Systems) in the fields of biological or medical applications has been in constant expansion. The development of so-called bio-MEMS (bio-micro-electro-mechanical systems) has become more and more popular (see "Packaging of Bio-MEMS: strategies, technologies, and applications, by T. Velten et al. in IEEE Trans. Advanced Packaging, vol. 28, no. 4, pp. 533-546, Nov. 2005). With bio-MEMS, next to the function of stimulation, the possibility for enhanced functionality of the electrodes is made possible. One example of this is feedback of data from a stimulated region, which may be with or without pre-processing.

From the packaging aspect, when implants have to be inserted in small organs with minimal invasive surgery, miniaturization obviously becomes a key requirement. As medical implants are rather complex systems including a broad variety of components (power source, transducers, control units...), special attention has to be paid to the packaging technology in order to be able to produce a compact device.

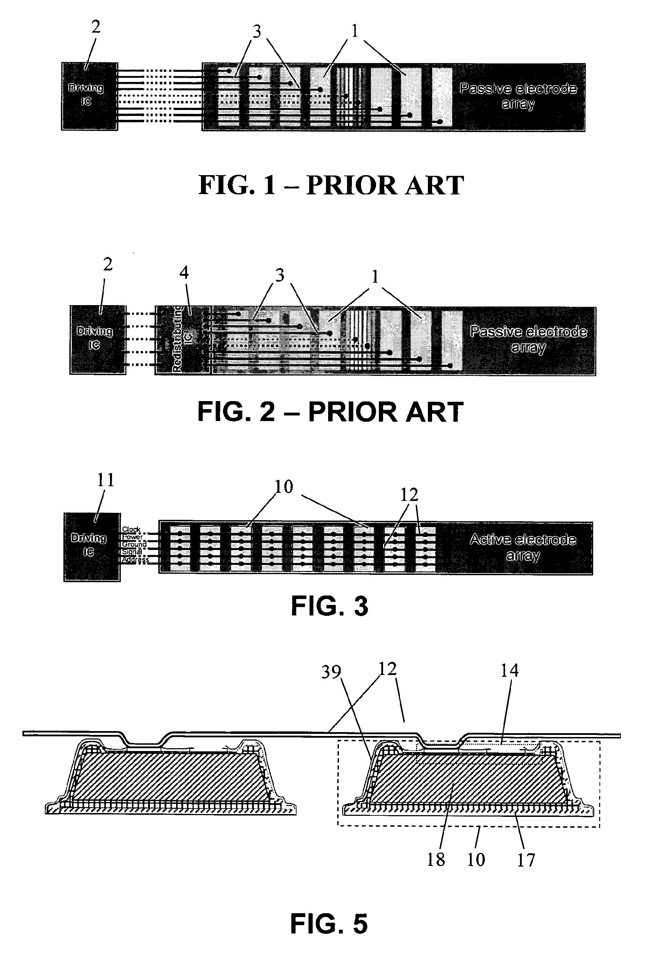

Regarding the current existing generation of implanted cochlear prostheses, all required electronics are located close to the skin, where a transmitter coil ensures the communication with an external module, together with the power supply (see "The past, present, and future of cochlear prostheses," F. Spelman in Engineering in Medicine and Biology Magazine, IEEE , vol.18, no.3, pp. 27-33, May/Jun 1999 and "Cochlear implants. Fundamentals and applications", by Graeme Clark, Springer-Verlag, New York, 2003). A long bundle of wires embedded in silicone and forming a "cable" links the electronics to passive electrodes which function as stimulation electrodes, are located inside the cochlea and of which between 16 to 22 may be present in the implant. In these implants, one individual wire is required for each of the electrodes. The length of this "cable" is approximately 20 cm. This may also be referred to as passive scheme or passive electrode array (see Fig. 1). It can be seen that the device comprises a number of electrodes 1 and a driving IC 2 for driving the electrodes 1. Each of the electrodes 1 therefore requires a wire 3 connecting the electrodes 1 with the driving IC 2. For such devices, current manufacturing techniques are based on manual assembly and therefore the manufacturing of such electrode arrays may be relatively expensive.

In recent work (see "An integrated position-sensing system for a MEMS-based cochlear implant," presented by J. Wang et al. at the IEEE International Electron Devices Meeting 2005, Washington DC, Dec. 2005; "A high-density electrode array for a cochlear prosthesis," by P. T. Bhatti et al. in TRANSDUCERS, Solid-State Sensors, Actuators and Microsystems, 12th International Conference on, 2003, vol.2, pp. 1750-1753, 8-12 June 2003 and "A 32-site 4-channel cochlear electrode array," presented by P. T. Bhatti et al. at the 2006 IEEE International Solid-State Circuits Conference, San Francisco, CA, Feb. 2006), a cochlear implant has been proposed in which use is made of an active chip 4 to drive an array of passive electrodes 1. This active chip 4 is located close to the electrode array, e.g. adjacent the electrode array as illustrated in Fig. 2. Using signal multiplexing, this limits the number of wires 3 required to connect that chip 4 to the driving IC 2 (power supply, speech processing unit...), allowing wider metal tracks and therefore lower resistivity. From the active chip 4, gold leads or wires 3 are redistributing the current to the passive electrodes 1 (see Fig. 2). This is an all-Silicon approach where the part implanted in the cochlea is made of a single piece of Si. This approach is very promising. However, this implant still needs a high number of wires 3, because still one wire 3 is required for each of the electrodes 1. If a larger number of electrodes 1, e.g. stimulation sites, is desired, the small dimensions of the cochlea, with a width varying from ~1000 µm down to ~200 µm, would impose a specific redistribution design of the connections or wires 3 to the passive electrodes 1, like a multilayer redistribution scheme. Furthermore, being made of one piece of Si, some concern can be raised regarding the reliability of the device. Due to the shape of the cochlea, an important bending of the implant is required and although the proposed device can be made very thin, it is still made of a brittle material. It also involves several dielectric materials whose integrity under bending may be affected. This can result in reliability and/or biocompatibility problems.

### Summary of the invention

It is an object of embodiments of the present invention to provide a packaged microelectronic device and a method for manufacturing such a packaged microelectronic device.

The packaged microelectronic device according to embodiments of the present invention may comprise less leads, wires or metal tracks than it comprises electrodes. Because of this, the number of electrodes that a packaged microelectronic device according to embodiments of the invention can comprise is not restricted by the amount of leads or wires required.

The packaged microelectronic device according to embodiments of the invention may comprise dense arrays of electrodes.

The above objective is accomplished by a method and device according to the present invention.

In an aspect, the present invention provides a packaged microelectronic device comprising at least one electrode comprising a chip embedded in a package, the chip comprising:
- a back electrode located at a first side of the chip, and
- electronic circuitry located at a second side of the chip, the second side being opposite to the first side,
wherein the back electrode is part of the package.

According to embodiments of the invention, the packaged microelectronic device may comprise a first number of electrodes and may furthermore comprise a second number of wires for connecting the electrodes to a drive circuit and to neighbouring electrodes of the microelectronic device. The first number may be higher than the second number.

The wires for connecting the electrodes to a drive circuit and to neighbouring electrodes of the microelectronic device may be formed by conductive tracks. According to embodiments of the invention, the conductive tracks may be formed by a stretchable interconnect, the stretchable interconnect comprising a horse-shoe shaped interconnection embedded in a silicone carrier.

The at least one electrode may be embedded in a biocompatible layer.

The biocompatible layer may comprises silicone or parylene.

According to embodiments of the invention, the packaged microelectronic device may furthermore comprise at least one conductive interconnect between the electronic circuitry and the back electrode. The at least one conductive interconnect may, together with the back electrode, form hermetically closed package of the chip.

The packaged microelectronic device may furthermore comprise a biocompatible layer on top of the at least one back electrode.

The at least one back electrode may be adapted for performing the function of sensing and/or stimulation.

The electronic circuitry may be adapted for providing sensing and/or stimulation signals from/towards the back electrode.

The microelectronic device may comprise a thin die or chip, which is packaged by and located within a hermetic package, wherein at least part of the external interface of the package comprises an electrically conductive surface, also referred to as back electrode, the electrically conductive surface being electrically connected to the thin die, the thin die being adapted for sending and/or receiving and processing signals from/to the electrically conductive surface.

Such a microelectronic device can comprise:
- a first electrically conductive layer forming an interaction or back electrode
- a thin die, the thin die having a front main surface comprising electronic circuitry comprising at least one front contact, and a back main surface, connected to the front main surface by a side surface, on top of the metal layer with the back main surface facing the layer of the first electrically conductive material,
- a first dielectric layer in between the back main surface and electrically conductive layer such that the thin die is electrically isolated from the first electrically conductive layer; the vertical projection of the die side surface not extending outside the first layer of electrically conductive material,
- a second dielectric layer on at least part of the side surface of the thin die,
- a second electrically conductive layer on the side surface of the thin die on top of the second dielectric layer, and on part of the front surface, such that at least one front contact is electrically connected to the first electrically conductive layer by means of the second electrically conductive layer.

A thin die may be a die with a thickness preferably lower than 100 µm, and preferably lower than 50 µm, and preferably lower than 30 µm and for instance between 10 µm and 20 µm.

The second dielectric layer can completely cover the side surfaces.

The second electrically conductive layer can cover the second dielectric layer on the side surface and the first electrically conductive layer and the second electrically conductive layer form a hermetically closed package of the thin die, except for a portion of the front surface.

The first dielectric layer can be a bonding layer.

According to embodiments of the invention, the packaged microelectronic device may comprise a passivation layer on the front surface of the thin die, such that the first electrically conductive layer, the second electrically conductive layer and the passivation layer form an hermetically closed package of the thin die.

The passivation layer can be biocompatible, or can be supplemented with an additional layer on top of the front surface, which is biocompatible.

The thin die can have a thickness of lower than 100 µm, lower than 50 µm, lower than 20 µm or lower than 10 µm.

The hermetic package can have a thickness of lower than 200 µm, lower than 100 µm, lower than 50 µm, lower than 20 µm or lower than 10 µm.

The first and the second electrically conductive layers can comprise or consist of biocompatible metals.

The first and the second dielectric layers can be patterned.

The thin dies can comprise CMOS structures, wherein the substrate is bonded such that the CMOS structures are facing the second carrier substrate.

The package can further comprise a silicone embedding layer.

The first electrically conductive layer, from which the back electrode are formed, can be adapted for providing sensing and/or stimulation.

The thin dies can advantageously be adapted for processing/providing sensing/stimulation signals from/towards the first electric layer, i.e. towards the back electrode.

The present invention also provides an interconnected device, comprising a series of N, with N at least 2, embedded thin dies or chips as described according to embodiments of the invention, the thin dies or chips comprising M, with M at least one, communication contact or wire on the front surfaces of the die, and at least M communication lines connected to the communication contacts adapted for receiving/transmitting networking signals from/to a control unit and/or other dies.

The communication lines can also serve as power supply lines.

In certain embodiments one communication contact is present for each die, and a single communication line is connecting these contacts.

In certain embodiments two communication contacts are present for each die, a first and a second communication contact, and only two communication lines are present connecting the first and the second communication contacts for each die respectively.
In certain embodiments X communication contacts are present for each die, a first , a second, ... and an Xth communication contact, and only X communication lines are present connecting the first, the second, ... and the Xth communication contacts for each die respectively.

The present invention also provides the use of a packaged microelectronic device according to embodiments of the present invention in medical or biomedical implants.

In another aspect the present invention provides a method for forming a packaged microelectronic device. The method comprises:
- providing a first carrier wafer comprising at least one back electrode,
- providing a wafer comprising electronic circuitry comprising at least one chip,
- attaching the first carrier wafer to the wafer to form a structure, such that the at least one back electrode is located at a first side of the structure and the electronic circuitry is located at a second side, opposite to the first side, of the structure,
- separating the chips from each other such that at least one electrode is formed, the electrode comprising a chip and a back electrode, and
- removing the first carrier wafer so as to release the at least one back electrode.

Providing a wafer comprising electronic circuitry may comprise:
- attaching a substrate comprising electronic circuitry to a second carrier wafer, and
- thinning the substrate so as to form the wafer comprising the electronic circuitry.

Providing a first carrier wafer comprising at least one back electrode may be performed by:
- providing a carrier wafer,
- onto the carrier wafer depositing a conductive layer, and
- patterning the conductive layer so as to form the at least one back electrode.

According to embodiments of the invention, the method may furthermore comprise providing conductive interconnects between the electronic circuitry and the back electrode.

According to embodiments of the invention the packaged microelectronic device may comprise a plurality of electrodes and the method may furthermore comprise providing wires for interconnecting the electrodes and for connecting them to a driving circuit.

Providing wires may be performed by providing conductive tracks, e.g. metal tracks.

Providing conductive tracks may be performed by providing stretchable interconnects, the stretchable interconnects comprising a horse-shoe shaped interconnection embedded in a silicone carrier.

According to embodiments of the invention, the method may furthermore comprise providing a biocompatible layer for embedding the at least one electrode. The biocompatible layer may, for example, be silicone or parylene.

The method may furthermore comprise providing a biocompatible layer on top the at least one back electrode.

Removing the first carrier wafer may be performed by removing a sacrificial layer present in between the first carrier wafer and the at least one back electrode. The sacrificial layer may, for example, be an aluminium layer.

The at least one back electrode may be adapted for performing the function of sensing and/or stimulation.

The electronic circuitry may be adapted for providing sensing and/or stimulation signals from/towards the back electrode.

The method according to embodiments of the invention may be a method for packaging thin dies or chips. The method may comprise:
- providing a first carrier substrate,
- producing a first electrically conductive layer on the first carrier substrate;
- providing a thin die, the thin die having a front main surface comprising at least one front contact and a back main surface, connected to each other by a side surface, on top of the metal layer with the back main surface facing the layer of the first electrically conductive layer, hereby also providing a first dielectric layer in between the back main surface and electrically conductive layer such that the thin die is electrically isolated from the first electrically conductive layer; the vertical projection of the die side surface not extending outside the first electrically conductive layer;
- producing a second dielectric layer on at least part of the side surface of the thin die
- producing a second electrically conductive layer on the side surface of the thin die on top of the second dielectric layer, and on part of the front surface, such that the at least one front contact is electrically connected to the first electrically conductive layer by means of the second electrically conductive layer.

The second dielectric layer can completely cover the side surface of the thin die.

A thin die may be a die with a thickness preferably lower than 100 µm, and preferably lower than 50 µm, and preferably lower than 30 µm and for instance between 10 µm and 20 µm.

The second electrically conductive layer can be produced such that it completely covers the second dielectric layer on the side surface and that the first electrically conductive layer and the second electrically conductive layer form a hermetically closed package of the thin die, except for a portion of the front surface.

The first dielectric layer may preferably be a bonding layer. It can be for instance silicone, a silicone based material or BCB (Benzo Cyclo Butene).

The method can further comprise producing a passivation layer on the front surface, such that the first electrically conductive layer, the second electrically conductive layer and the passivation layer form an hermetically closed, package of the thin die.

The package can further be embedded in an embedding material as for instance a silicone layer, a layer comprising silicone based material or a BCB layer.

The first carrier substrate may preferably be a temporary carrier substrate; the method can further comprise a step of producing a selectively removable sacrificial layer on the first carrier substrate before producing a first electrically conductive layer on the first carrier substrate, on top of the sacrificial layer.

The sacrificial layer can then be removed at a later stage, thereby releasing the produced structure.

In certain embodiments the first carrier substrate can be part of the package ; it can be such that it allows interaction between the first electrically conductive layer and the environment external to the package. In an other view it is not part of the package, but it remains on the package - hereby not preventing interaction between the first electrically conductive layer and the environment external to the package, on the non bonded side of the first carrier substrate.

For biomedical applications, the first and the second electrically conductive layers are preferably biocompatible metal layers. They can comprise platinum, platinum-iridium, iridium, titanium, alloys of the foregoing and other metals.

The passivation layer can also be a biocompatible passivation layer. It can be for instance silicone, a silicone based material, BCB or preferably parylene C.

In typical embodiments the first and the second dielectric layers can be patterned.

The thin die can preferably be provided by an ultra thin die stacking process, comprising:
- bonding a substrate to a second temporary carrier substrate;
- thinning of the substrate on its non bonded side;
- providing the first temporary carrier substrate, comprising the sacrificial layer and the first electrically conductive layer;
- bonding the thinned wafer to the electrically conductive layer carrying substrate;
- singulating or separating dies of the electrode carrying substrates at wafer level;
- thin film integration of active dies at wafer level;

This substrate can comprise CMOS structures, and the substrate can be bonded such that the CMOS structures are facing the second carrier substrate.

The first electrically conductive layer can be adapted for providing sensing and/or stimulation.

In advantageous embodiments the thin dies are adapted for processing, receiving and/or providing sensing or stimulation signals from or towards the first electric layer.

The present invention also provides a composite stretchable electrical interconnect comprising:
- a stretchable support or embedding material;
- an electrically conductive line or interconnection extending between a first and a second end point, in physical contact with and being supported by the stretchable support or embedding material;
wherein the electrically conductive line has a meandering shape corresponding to a repetition of a basic shape element, the basic shape element being such that, when following the line along its trajectory within a basic shape element in the direction from the first end point to the second end point, the distance to the end point is temporarily increasing for at least one part of the trajectory.

The electrically conductive line may comprise a number of sub-lines. This provides the advantage that for an equivalent global deformation of the stretchable support, the local strain in one of electrically conductive the sub lines is lower than the strain in the electrically conductive line in the case where no sub lines are used, a lower local strain being advantageous regarding reliability aspects.

The basic shape element can be horse-shoe like.

The electrically conductive line can advantageously be positioned along a straight line between a first end point and a second end point, the straight line being oriented along a direction which stretching of the composite stretchable electrical interconnect is expected to occur during normal use.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 and 2 show microelectronic devices according to the prior art.
Fig. 3 schematically shows a microelectronic device according to embodiments of the invention.
Fig. 4 schematically illustrates a process flow according to embodiments of the invention.
Fig. 5 is a schematic cross section of a 1 D electrode array formed by a process according to embodiments of the invention.
Fig. 6 illustrates Pt patterns obtained by lift-off, meanders test structures used for determination of metal sheet resistance (left part) and patterned Pt electrodes as on an electrode carrier wafer (right part).
Fig. 7 compares dies formed by standard DRIE process (left part) and by an optimized process to achieve sloped sidewalls (right part, before photoresist removal).
Fig. 8 illustrates a stretchable interconnection.
Fig. 9 shows a force-deformation curve for a 24 mm long stretchable interconnect in a 35 µm thick silicone carrier.
Fig. 10a to 10z illustrate subsequent steps in a method according to embodiments of the invention.
Fig.11 and 12 shows microelectronic devices according to embodiments of the invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

The present invention provides a packaged microelectronic device and a method for manufacturing such a packaged microelectronic device.

The microelectronic device comprises at least one electrode comprising a chip embedded in a package, the chip comprising:
- a back electrode located at a first side of the chip, and
- electronic circuitry located at a second side of the chip, the second side being opposite to the first side,
wherein the back electrode is part of the package.

The microelectronic device according to embodiments of the present invention comprises electrodes, e.g. stimulation or sensing sites, formed by chips or dies with reduced size. This is because the electronic circuitry are located at one side of the chip and the active electrode, e.g. stimulation or sensing electrode, also referred to as back electrode or interaction electrode, is located at another side of the chip, opposite to the one side. The main electronics part is furthermore directly connected to the active or back electrodes of the device, following a concept that is called "active electrode array". The microelectronic device according to embodiments of the invention comprises a number of electrodes 10 and a driving IC 11 for driving the electrodes 10. This is schematically illustrated in Fig. 3. Each of the electrodes 10 is formed of a chip comprising electronic circuitry and a back electrode. This will be described in detail hereinafter. In order to improve the reliability, the whole structure can be embedded in an embedding material such as a silicone material, a silicone based material or BCB (Benzo Cyclo Butene).

In the microelectronic device according to embodiments of the invention, each electrode 10 is a "real" chip whose backside may be covered with biocompatible metal, and which comprises a back electrode on that backside acting as active electrode, e.g. stimulation or sensing site. Using, for example, a bus architecture and multiplexer-demultiplexer IC's, those active electrodes 10 are interconnected via a limited number of leads or wires 12. In the example given in Fig. 3, only six wires 12 are required for driving all of the electrodes 10. When addressed, the chip delivers current to the addressed or selected active electrode, e.g. stimulation or sensing site. It can be seen that where the number of leads 12 required for passive electrodes is directly proportional to the number of electrodes, the active electrodes concept according to embodiments of the invention only requires a limited number of leads 12, which is independent from the number of electrodes 10 in the microelectronic device. Basically, in case of, for example, stimulation, it is already possible to drive a large number of electrodes with only five lines connecting the driving IC 11 with the electrode array (clock, power, ground, signal and address, see Fig. 3). Then only one extra lead 12 is required between the electrodes 10 for signalling.

Hence, according to embodiments of the invention, the number of wires or leads 12 for driving and connecting the electrodes 10 is less than the number of electrodes 10 present in the microelectronic device. The fact that the number of wires 12 required can be kept low allows to increase the number of electrodes 10 present in the microelectronic device without making the device more complex. Increasing the number of electrodes 10 will increase the resolution of the microelectronic device. According to embodiments of the present invention, the number of electrodes 10 can in principle be altered up to any number of electrodes 10. The number of electrodes that can be incorporated in the microelectronic device according to embodiments of the invention may only be restricted by the technology used.

The method according to embodiments of the present invention aims at producing microelectrode arrays, using thin-film technology. This allows low dimensions, with a micrometer control, and a high reproducibility. The use of this technology may also lead to reduced costs compared to currently used production processes.

The method and microelectronic device according to embodiments of the present invention may be used for any application where a packaged device is required and where complexity of the device and costs should be kept low. The method and microelectronic device according to embodiments may be advantageously used for medical or biomedical devices and especially for medical or biomedical implants, such as for example cochlear implants. Beside cochlear implants, which make use of 1 D electrode arrays, another application may retina implants, where 2D electrode arrays are required. To some extent, such 2D electrode arrays bring similar challenges than cochlear prostheses (see "Microelectronic Packaging for Retinal Prostheses," by D. C. Rodger et al. in IEEE Engineering in Medicine and Biology Magazine, vol. 24, no. 5, pp. 52-57, Sept.-Oct. 2005) and the microelectronic device according to embodiments of the present invention may be used for retina implants.

It has to be understood that the method and microelectronic device according to embodiments of the present invention are not limited to the above described examples and to the examples used for describing the invention.

Furthermore, it has to be noted that wherever in the description or claims is referred to interconnect, tracks, conductive tracks, wires or communication contact, the same thing is meant, i.e. means for connecting the electrodes to each other and to a driving circuitry.

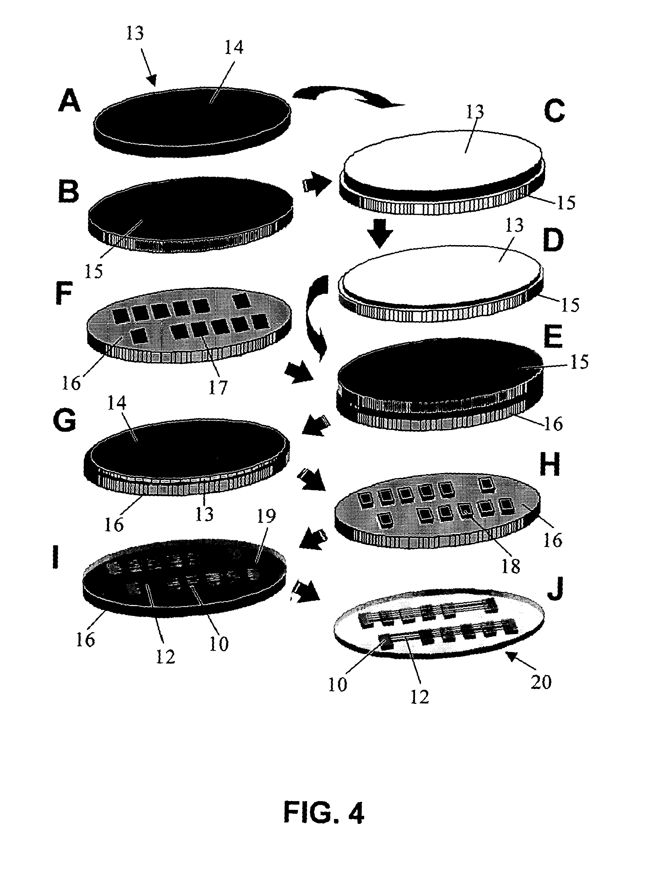

Fig. 4 schematically illustrates a possible sequence of different steps required for forming the microelectronic device 20 according to embodiments of the invention. Using wafer to wafer bonding, an active wafer 13 (see step A), or part of an active waver 13, is assembled face down to a carrier wafer 15 (see step B). With active wafer 13 is meant a wafer 13 comprising an electronic circuitry 14, also referred to as active circuitry, for, when in use, driving the electrodes 10 of the microelectronic device 20. With assembling face down is meant that the active wafer 13 is bonded to carrier wafer 15 with the electronic circuitry 14 facing carrier wafer 15. The result of this wafer to wafer bonding is illustrated in step C. With back grinding and polishing, the active wafer 13 may then be thinned down from the backside, i.e. from the side opposite to the side where the electronic circuitry 14 is located, to a desired thickness (see step D). After this step, the resulting stack, i.e. carrier wafer 15 with thinned active wafer 13, is aligned and bonded (see step E) to another carrier wafer 16 (see step F) on which back electrodes 17, which will later serve as active electrodes, e.g. for stimulation or sensing, are formed. Carrier wafer 15 may then be debonded (see step G) from active wafer 13 for exposing the electronic circuitry 14 on the active wafer 13. Next, the active wafer 13 may be patterned (see step H) to form single dies or chips 18. This patterning may be done such that for each back electrode 17 on carrier wafer 16 a die or chip 18 is provided. Patterning may be done by using Deep Reactive Ion Etching (DRIE). A die or chip 18 together with its corresponding back electrode 17 then forms an electrode 10. Then, further packaging can take place using thin film technology. The individual chips 18 can be insulated, coated, connected and/or embedded (see steps I, indicated with reference number 19). As a last step, carrier wafer 16 may be debonded. Step J illustrates the finished microelectronic device 20.

In certain embodiments as described above, the disclosed processes may use temporary carrier substrates 15, 16. This results in the realization of the whole process at Wafer Level (WL), the final step being the release of the manufactured structures by removal of a sacrificial layer (see further).

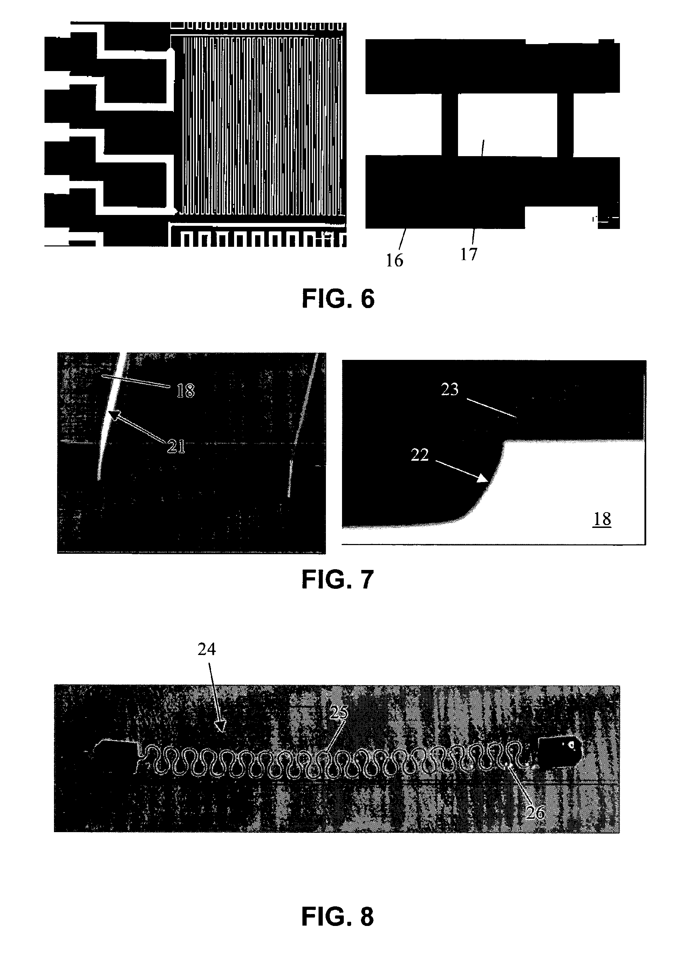

Several process steps of the above described flow are based on an Ultra Thin Chip Stacking (UTCS) process (see "Ultra thin electronics for space applications," by O. Vendier et all. In Electronic Components and Technology Conference, 2001, Proceedings, 51st , vol., pp. 767-771, 2001, European patent application EP 1 041 620, US patents US 6,506,664 and US 6,730,997). In addition, novel technology was developed for removal of the sacrificial layer so that individual, flexible electrodes are released. Fig. 5 shows a schematic cross section of a 1 D electrode array, formed by the process as described above, after the release of carrier wafer 16 (step J in Fig. 4) according to the process flow as described above.

The above described process flow may have the advantage of good placement accuracy and throughput (wafer to wafer bonding compared to flip chip die bonding). However, a drawback of the above described flow is that, for low density arrays, i.e. arrays comprising only a few electrodes 10 on a large wafer 13, a significant amount of the active wafer 13 may be wasted when it is etched to form the chips or dies 18. Therefore, when very dense array of electrodes 10 are not required, the above described process flow may be slightly modified, based on die-to-wafer assembly, as known by a person skilled in the art. This also allows combining IC's of various types in one microelectronic device 20.

Whereas silicone embedding is frequently used in biomedical implants, integration of elastomeric materials in MEMS devices is rather uncommon. The use of silicone as a dielectric and/or embedding material may be advantageously used in the process according to embodiments of the present invention because it combines flexibility and stretchability, which may improve the reliability of the microelectronic device 20. Flexibility and stretchability are important properties of microelectronic devices 20 which have to be implanted in a human body because they are subject to repeated deformation (body movement, heartbeat, ...) which could cause fatigue failure of at least part of the microelectronic device 20 after some time. The silicone can also be patterned e.g. by using a plasma. Dry etch patterning of the silicone based on a fluorine plasma has been demonstrated by M. Vanden Bulcke et al. in "Introducing a silicone under the bump configuration for stress relief in a wafer level package," Electronics Packaging Technology, 2003 5th Conference (EPTC 2003) , pp. 380-384, 10-12, Dec. 2003. In this document, etch rates close to 1 µm/min were obtained.

For medical or biomedical applications, beside the embedding material, which may be a dielectric material, other materials involved in the fabrication of the microelectronic device 20 and which, after manufacturing, are at the outside of the device 20, have to be customized in implant applications keeping biocompatibility in mind. As a metal for forming e.g. back electrodes 17 or interconnects, Pt may be chosen. This material is biocompatible and commonly used for forming, for example, stimulation electrodes. In the active electrode array, Pt may be used as an electrode surface, as capping material for the chip 18 and as metal interconnect (see further). However, Pt is not easily etched and therefore a lift-off technique was developed. For the purpose of illustration, Fig. 6 shows Pt patterns obtained by lift-off. The left part of Fig. 6 shows meander test structures used for determination of metal sheet resistance and the right part of Fig. 6 shows patterned Pt electrodes 17 formed on a carrier wafer 16 and intended to function as back electrodes 17 in the microelectronic device 20 after manufacturing. A compromise was reached between a thick Pt layer which is more difficult to process by lift-off and a thin layer whose electrical resistance would have been too high. Final selection was a layer thickness of 1 µm, for a sheet resistance of ~170 mOhm/square (or less than 100 Ohm resistance for a 50 µm wide and 30 mm long track).

In addition to silicone, another frequently used biocompatible material, parylene C, may also be applied, e.g. for encapsulating Pt interconnects to improve their mechanical resistance. Parylene C may also used as additional capping material for the dies 18. Adhesion of the Pt and silicone to the parylene may be increased by an O₂ plasma prior to deposition.

Final release of the temporary carrier wafer 16 as described with respect to Fig. 4, step J, results in free-standing flexible devices. This step may require the selection and application of a suitable sacrificial layer (see further). An important condition for this layer is that it must withstand the whole process, but should still be easily removable when necessary at the end of the manufacturing process. SiO₂ was considered to be used as a sacrificial material, but etching of this material occurs in vapor HF (see "A comparison between wet HF etching and vapor HF etching for sacrificial oxide removal," by A. Witvrouw et al. in Proc. SPIE Micromachining and Microfabrication Process Technology VI, Vol. 4174, pp. 130-141, Sept. 2000 and "HF etching of Si-oxides and Si-nitrides for surface micromachining," by B. Du Bois et al. in Proc. of the Sensor Technology Conf. 2001, pp. 131-136, 2001), which is not suitable for use in the manufacturing process according to embodiments of the present invention as it might be removed during the manufacturing of the microelectronic device 20. A thermally releasable sacrificial material was another option. Different types of materials which can be used as sacrificial material exist, but none of them is offering stability during e.g. silicone cure (250 °C), together with a convenient release leaving a residue free surface.

According to embodiments of the invention, an Al layer may be used as a sacrificial layer. Removal of the Al layer after finishing the microelectronic device may then be based on an enhanced anodic dissolution of the Al layer when immersed in a sodium chloride solution while a positive potential is applied to the Al layer. An advantage of such technique is that the release takes place at room temperature in a neutral saline fluid and that it avoids the use of acid or any other aggressive solution.

Separation of the different dies or chips 18 (see Fig. 4, step H) may be done by standard DRIE. In this case, after etching, the formed sidewalls 21 of the chips 18 may be vertical (see Fig. 7, left part), i.e. may be oriented in a direction, when carrier wafer 16 is lying in a plane, substantially perpendicular to the plane of the carrier wafer 16. This makes subsequent steps (e.g. provision of dielectric materials) more complex, but not impossible, because of difficulties with sidewall coverage. In order to facilitate sidewall coverage, the DRIE process has been optimized by using adequate gas compositions and ratio, power and pressure. The gases used can be SF₆/C₄F₈/O₂, for instance in a ratio 8:3:2. The process resulted in the production of ~65° sloped sidewalls 22 (see Fig. 7, right part). It has to be noted that in the right part of Fig. 7 the photoresist 23 used during etching is still present on the die 18.

After release of carrier wafer 16, the dies can be handled individually and both mechanical and electrical measurements are possible. Electrical measurement of a wire 12, also referred to as metal track, embedded in the silicone carrier has been carried out. This measurement was performed on a flat sample. The sample has then been rolled up in order to form a 360° loop and new resistance measurement has been performed. Results showed that even when completely bent, the embedded track 12 is still conductive.

As suggested when mentioning the use of silicone, one advantageous feature of this material is its stretchability. However, the metal wires 12 used as interconnects between the electrodes 10 (see further) in the microelectronic device 20 are not stretchable. According to embodiments of the present invention, the possibility to make the interconnects stretchable has also been studied. The outcome of this work is that stretchability may be achieved by designing the wires 12 as a metal interconnect 24 in a meander shape rather than a straight shape. This is illustrated in Fig. 8 which shows a "horse-shoe" shaped interconnection 25 embedded in a silicone carrier 26.

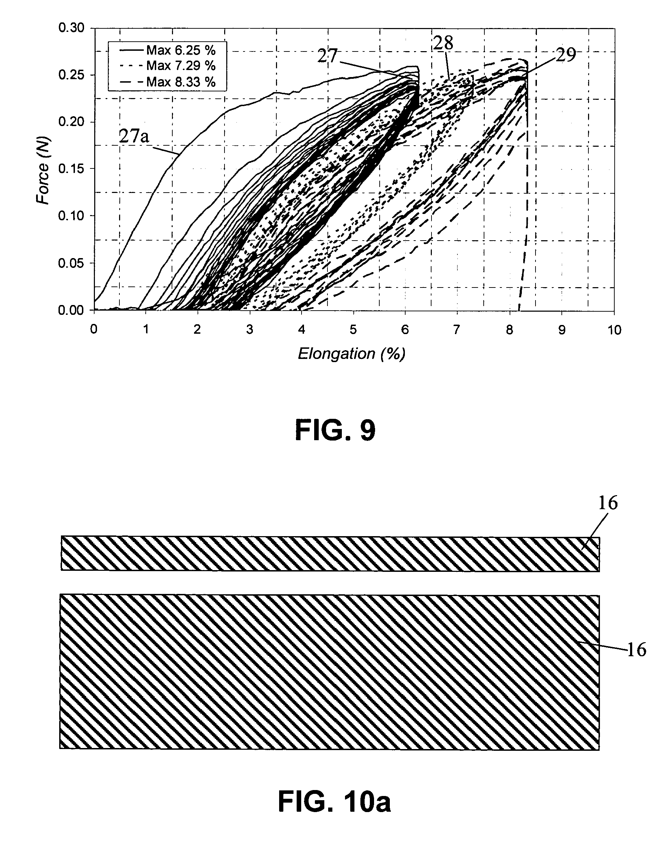

Fig. 9 shows a force-deformation curve for a stretchable interconnect 24 comprising a 24 mm long horse-shoe shaped interconnection 25 in a 35 µm thick silicone carrier 26 which has been submitted to repeated deformation. The tensile test was set up to stop elongation at a fixed deformation and bring the sample back to its original length, still recording the force. A first series of tests was done up to 6.25 % elongation (curve 27). Line 27a of this curve 27 in Fig.7 corresponds to plastic deformation of the stretchable interconnect structure 24. For subsequent deformation cycles, an elastic hysteresis is observed. This elongation to 6.25 % was repeated twenty times. The system was then set to extend the stretchable interconnect structure 24 to 7.29 % of its original length (curve 28). The cycle was repeated ten times and, again, an elastic hysteresis is observed. Finally, the deformation cycle of the stretchable interconnect structure 24 was extended to 8.33% (curve 29) and, after ten additional cycles, the sample broke. This shows that the silicone structures realized with thin film can be made stretchable, even with a thickness of ~35 µm. To some extent, repeated deformation can be applied without breaking the silicone. Simple electrical resistance measurements performed after stretching experiments showed that the embedded horse-shoe shaped interconnection 25 was still conductive.

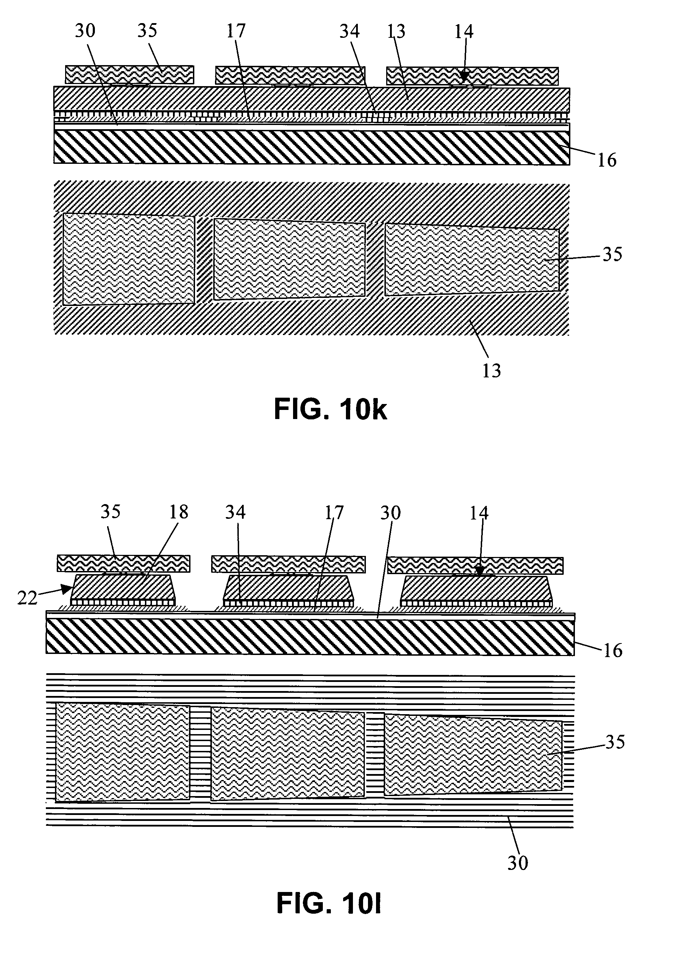

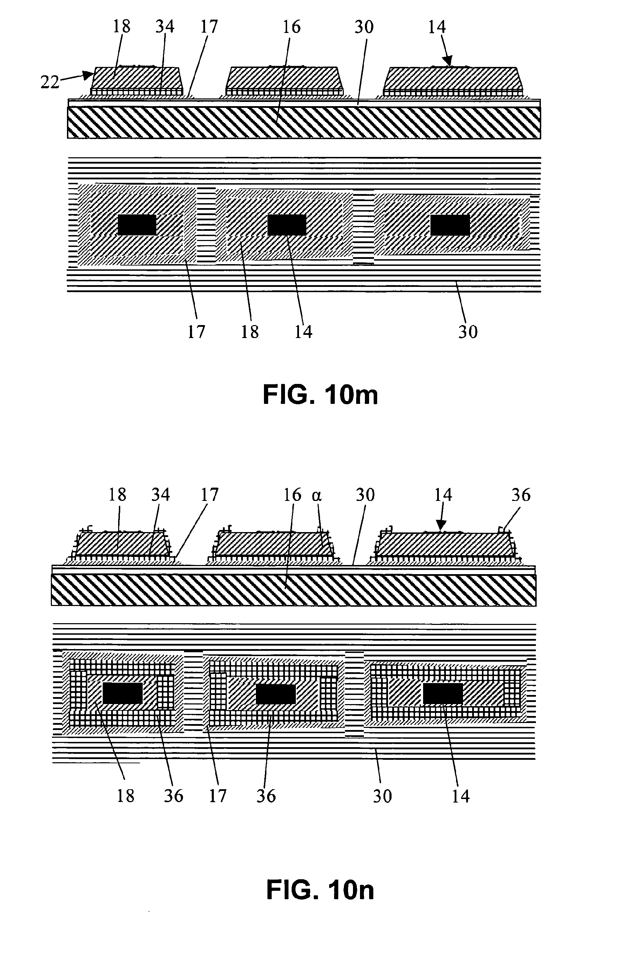

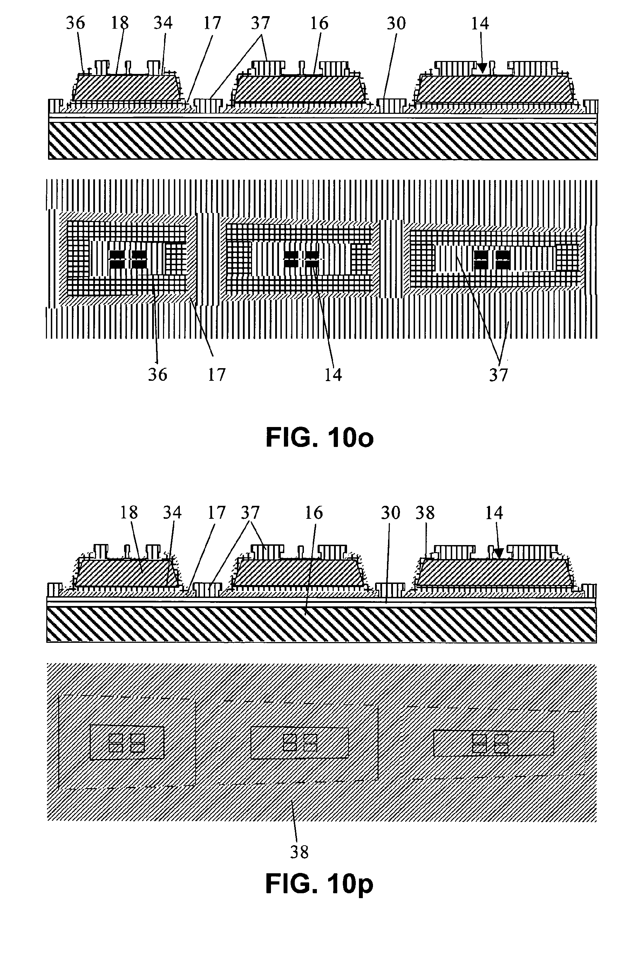

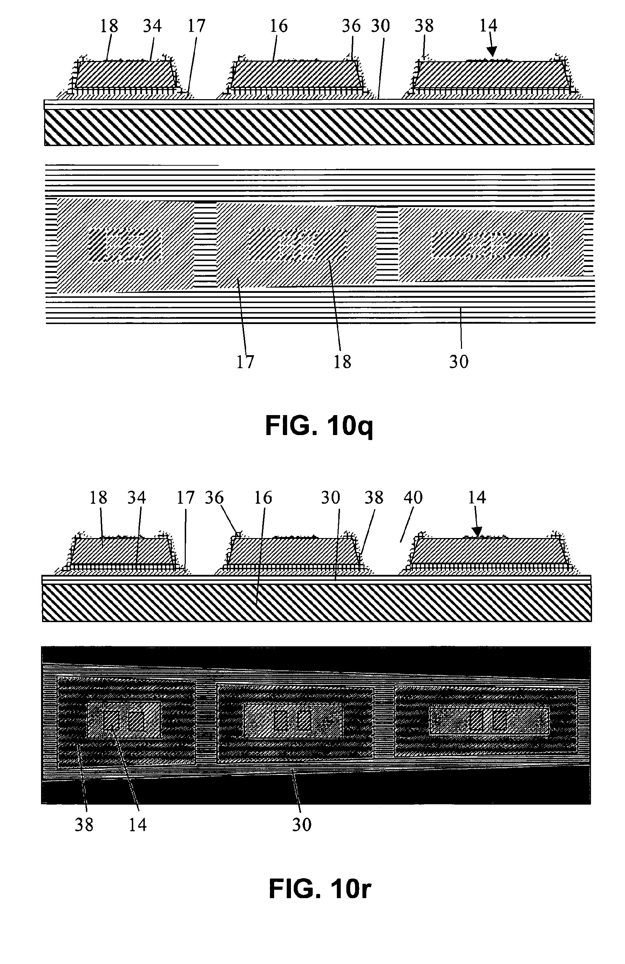

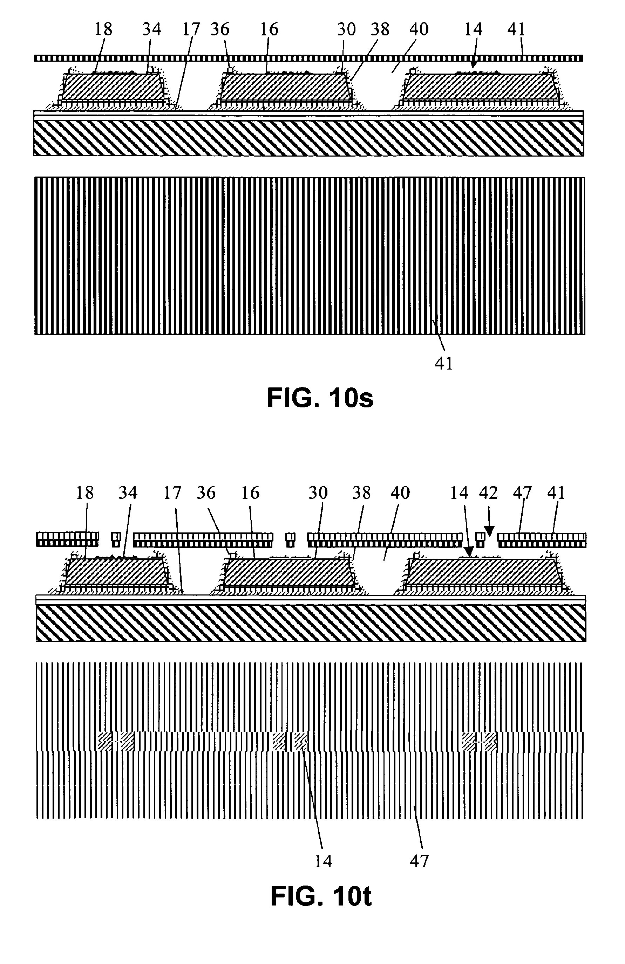

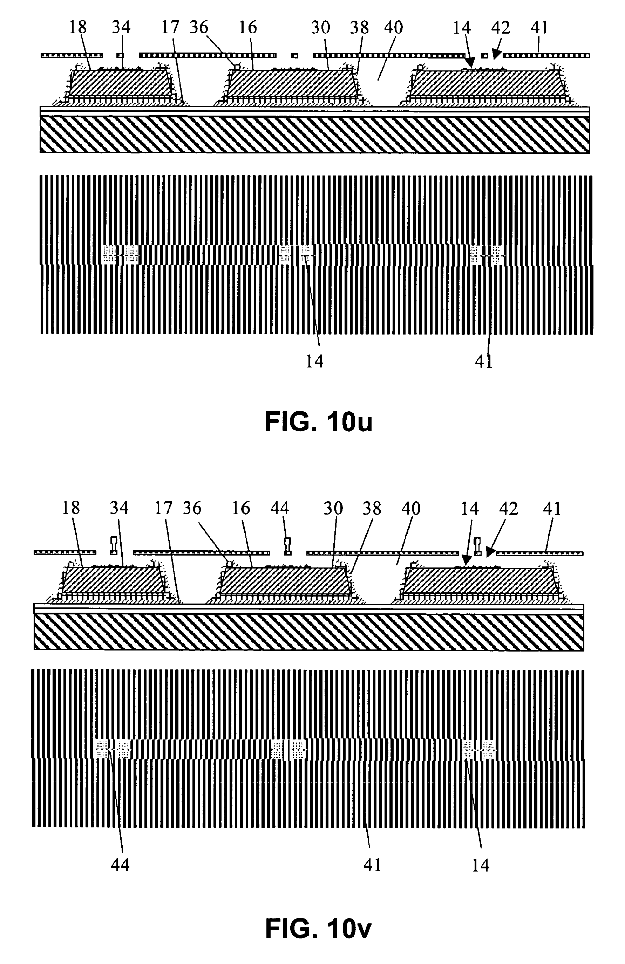

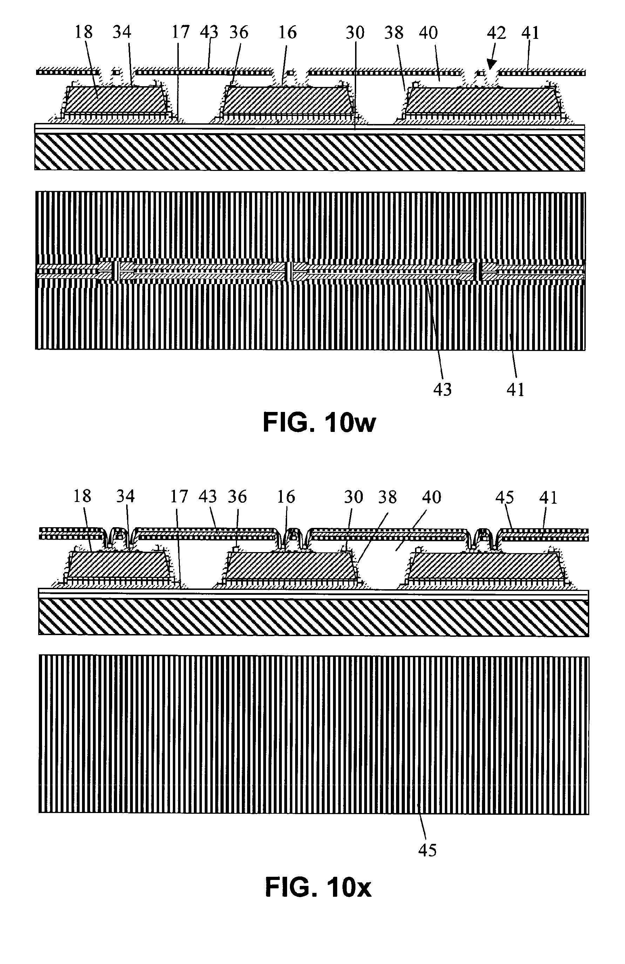

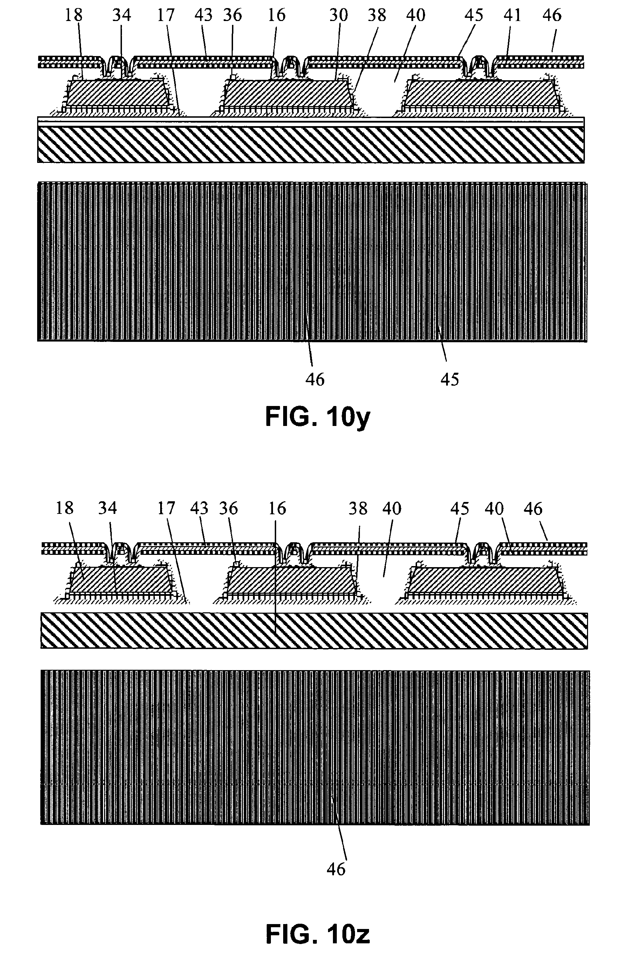

Hereinafter, a detailed description will be given for the different steps of the process flow described above by using Figures 10a to 10z. It has to be noted that this is only for the ease of explanation and that it is not intended to limit the invention in any way. The process flow may, according to embodiments of the invention, also have other sequences or may use other materials. The Figures 10a to 10z illustrating the different process steps are such that they comprise two parts. The upper part represents a side view and the lower part represents a top view of the microelectronic device 20 at the respective state of processing.

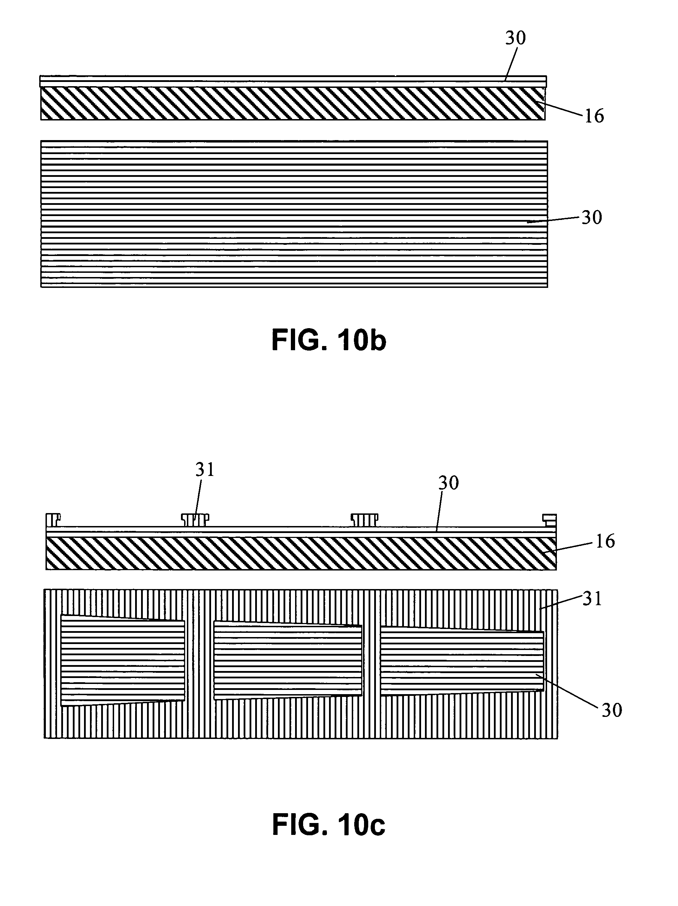

First a first carrier wafer 16, also referred to as first temporary carrier wafer or carrier substrate, is provided (see Fig. 10a). This may, for example, be a Si wafer. A sacrificial layer 30 is deposited on top of the first temporary carrier wafer 16 (see Fig. 10b). This sacrificial layer 30 may preferably be removed at the end of the process, thereby releasing the free-standing final structure. The sacrificial layer 30 may comprise aluminum, but may also be any other suitable material which is able to withstand the whole process while still being able to be removed at the end of the process, e.g. by etching, by dissolving or by evaporation. The sacrificial layer 30 may preferably have a thickness of 1-2 µm, but thicker layers may also be used possibly with addition of topography or a pattern in this layer that would lead to formation of textured back electrodes 17 during subsequent steps.

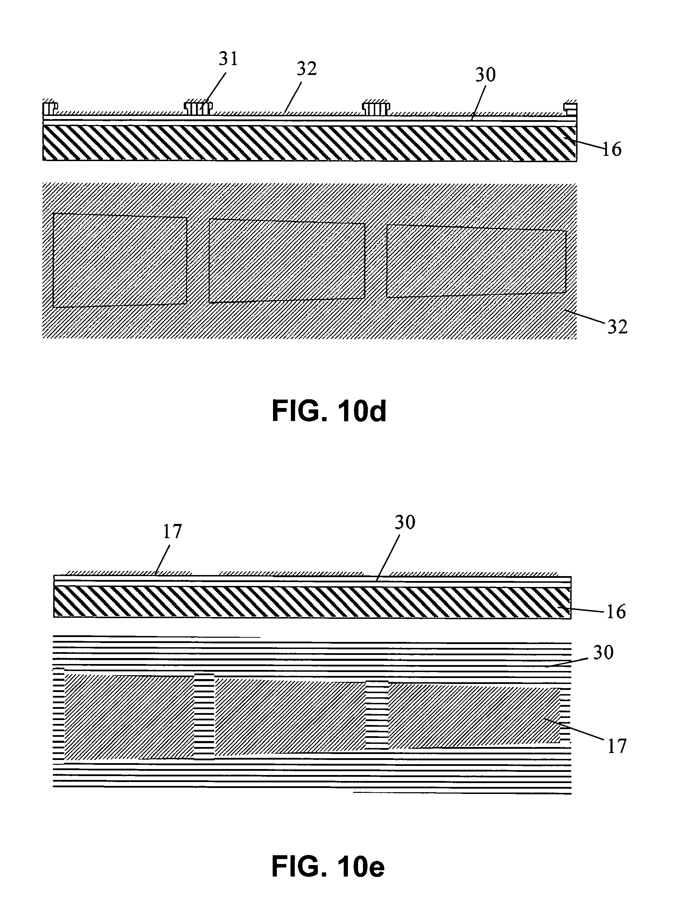

In a next step which is illustrated in Fig. 10c definition of the back electrodes 17, also referred to as bottom electrodes 17, may be performed. The back electrodes 17 may preferably be made of platinum but any other suitable biocompatible metal can be used. The formation of the back electrodes 17 can be achieved by electroplating (electrolytic or electroless), by lift-off technique or by etching a metal layer. In the present example, formation of the back electrodes 17 may be performed by lift-off. For lift-off, a photo resist layer 31 can be applied and patterned accordingly to a required final electrode structure (see Fig. 10c). Next, a metal, in the example given platinum, layer 32 may be deposited. The metal layer 32 can be deposited onto the whole wafer as can be seen from Fig. 10d. The metal layer 32 may then be patterned to form the back electrodes 17. This may be done by removing the patterned photoresist layer 31 e.g. by dissolution in a suitable solvent, through which only metal that was deposited where the first carrier wafer 16 was exposed remains, thereby forming the back electrodes 17 (see Fig. 10e) and the metal which was on the patterned photoresist layer 31 is lifted off together with the photoresist material.

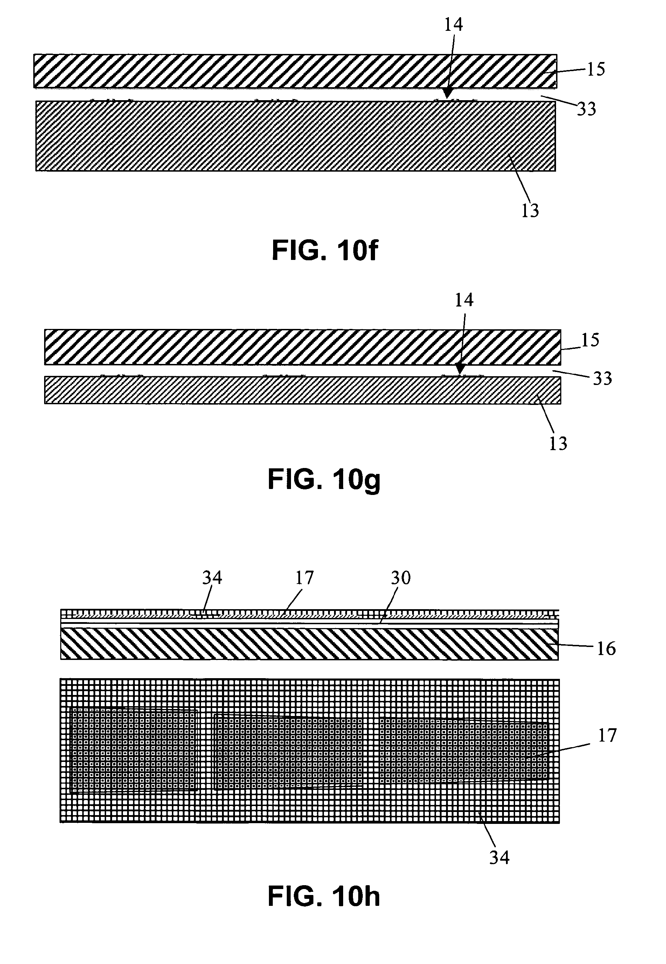

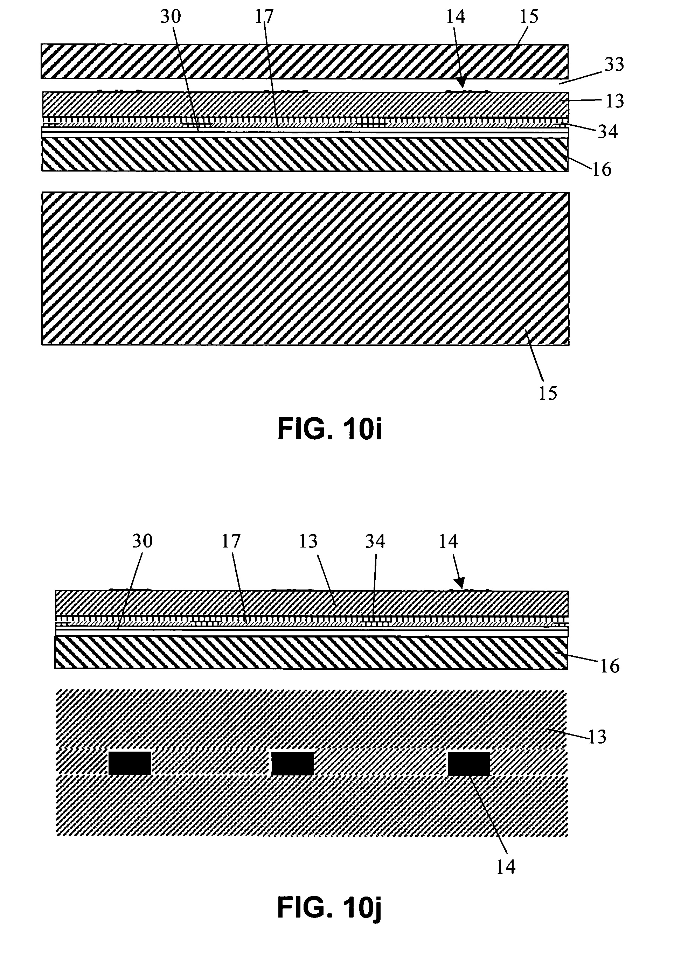

As already explained above, electronic circuitry 14 is to be provided for forming the electrodes 10 of the microelectronic device 20. The electronic circuitry 14 may be formed on a thin substrate, also referred to as active wafer 13, with a thickness of between 10 and 50 µm, preferably between 10 and 20 µm. For the ease of handling this thin substrate, first the circuitry 14 may be formed on a thicker substrate (see Fig. 10f) and may then be placed on a second carrier wafer 15, also referred to as second temporary carrier wafer. This can be achieved by die-to-wafer assembly, when only a limited number of electrodes 10 is to be formed, or by wafer-to-wafer assembly, as described above. The carrier wafer 15 may, for example, comprise silicon. The active wafer 13 comprising the electronic circuitry 14 may be bonded to the second carrier wafer 15 by a suitable bonding material 33, e.g. wax (see Fig. 10f), also referred to as temporary bonding material 33 because it will have to be removed further on during the manufacturing process (see further). The active wafer 13 may then be thinned down to a desired thickness (see Fig. 10g).

The active wafer 13 bonded to carrier wafer 15 can then be placed onto the first carrier substrate 16 comprising the back electrodes 17. Therefore, that side of the active wafer 13 comprising the electronic circuitry 14 is bonded to that side of carrier substrate 16 comprising the back electrodes 17. In order to ensure good adhesion, a gluing material 34 may be applied between carrier substrate 16 and the active wafer 14. This material can be silicone, a silicone based material, BCB or any other suitable material 34 ensuring a good bonding of the two parts together. This material 34 can be deposited onto carrier substrate 16 comprising the back electrodes 17 (as illustrated in Fig. 10h) and/or on the active wafer 13 comprising the electronic circuitry 14. The two "stacks" can then be assembled together (see Fig. 10i).

Removal of the temporary bonding material 33 then enables the release of the temporary carrier wafer 15 through which the electronic circuitry 14 on the active wafer 13 is exposed and becomes accessible (see Fig. 10j). Optionally, a cleaning step may be performed to ensure complete removal of the temporary bonding material 33. This cleaning step may comprise any method known by a person skilled in the art suitable for removing residues of the bonding material 33 without damaging the underlying active wafer 13.

In case of wafer-to-wafer assembly, removal of the material, e.g. Si, in between neighbouring parts of the electronic circuitry 14 may be required in order to separate the different dies or chips 18. Therefore an etch mask 35 may be provided on top of the electronic circuitry 14 (see Fig. 10k). This etch mask 35 may be any suitable etch mask known by a person skilled in the art, such as e.g. a photoresist. The excess material of the active wafer 13, e.g. Si, can then be removed by way of, for example, wet or dry etching, thereby releasing the individual dies or chips 18 (see Fig. 101). Each chip 18 may have a thickness of lower than 100 µm, lower than 50 µm, lower than 20 µm or lower than 10 µm. Preferably, removal of the material of the active wafer 13, e.g. Si, may preferably be done by etching by means of the modified DRIE process as described earlier, such that the chips 18 formed may have a sloped sidewall 22 as discussed above. However, this is not necessarily so but is preferred in order to obtain a good coverage of the sidewalls 22. After formation of the chips 18, the mask 35 may be removed according to any suitable technique known by a person skilled in the art (see Fig. 10m).

Up till now, a microelectronic device is formed comprising electrodes 10 comprising electronic circuitry 14 at a first side of the device and a back electrode 17, also referred to as active electrode and suitable for forming a sensing or stimulation site of the device, at a second side opposite to the first side of the device. Through this, the microelectronic device 20 according to embodiments of the invention may have a reduced size with respect to prior art devices. This is because in prior art devices the active electrodes and the electronic circuitry are located on a same side of the device. By making the active electrode 17 at an opposite site of the side where the electronic circuitry 14 is formed, the microelectronic device 20 according to embodiments of the present invention can be made more compact compared to prior art devices. This is an important property, especially when the microelectronic device 20 is used as a medical or biomedical implant. Moreover, as will be discussed later, the back electrode 17 forms part of the packaging of the device 20.

Because the back electrode or active electrode 17 is formed on an opposite side of the side where the electronic circuitry 14 is located, a contact is required from at least one top contact pad of the electronic circuitry 14 to the back electrode 17. Furthermore, an insulation of the chip sidewalls 22 from that contact is required. Insulation can be obtained by providing an insulating material 36 to the sidewalls 22 of the chips 18 (see Fig. 10n). Depending on the slope of the sidewalls 22 and on the required final thickness of the microelectronic device 20, different coating techniques may be used such as e.g. spin coating, spray coating, .... Spin coating may preferably be used for applying insulating material to sidewalls 22 with a moderate sidewall slope making an angle α with a base surface of the carrier wafer 16 looked upon from within the die of lower than 45°, preferably lower than 40° in combination with a smaller thickness of the chip or die 18 of lower than 50 µm, preferably lower than 20 µm, more preferably lower than 10 µm and most preferably lower than 5 µm. For steeper sidewalls (angle of around 45°) and/or thicker dies (thicker than 50 µm), spray coating may be used. This insulating material 36 can partially embed the chip.

Next, a patterned photoresist layer 37 may be provided as is illustrated in Fig. 10o. This may be done by any known suitable technique. A metal layer 38 may then be deposited over the complete surface of the device obtained up till now (see Fig. 10p). The metal layer 38 may be provided by any suitable technique known by a person skilled in the art. Removal of the patterned photoresist layer 37 leads to the configuration as illustrated in Fig. 10q. The metal layer 38 combines a function of providing electrical connection of a pad of the chips 18 to the back or active electrode 17 with a function of encapsulation of the chips 18.

In a next step, optionally, an additional hermetic biocompatible layer 39, as illustrated in Fig. 5 but not in this example, could be inserted at this stage in order to offer a full bio compatible hermetic encapsulation of the chip 18. The chip 18 can be contacted through this biocompatible layer 39 via the back electrode 17.

After encapsulation, a layer of soft biocompatible material 40 can be applied in order to ensure a perfect biocompatible and hermetic encapsulation of the silicon chips 18 (see Fig. 10r). The soft biocompatible material 40 may be a stretchable material and may be applied and patterned according to the desired final shape of the device 20. Patterning can be achieved by lithography in the case of e.g. a photosensitive material, but it can also be obtain by etching, e.g. by applying a dry etch. An effect of the application of this layer may be the reduction of the height difference as some planarization effect.

In a further step, a layer 41 of strengthening material may be applied in order to enforce the strength of wires or tracks 12 that will be formed subsequently. These wires 12 are for connecting the chips 18 to a drive circuitry 11 and to subsequent chips 18. Such material may, for example, be parylene.

In order to interconnect the chips 18, their contact pads have to be opened. One possibility to achieve this is to use dry etching of the material through a patterned photo resist mask 48 to form holes 42 (see Fig. 10t). After forming the holes 42, the photoresist mask 48 may be removed (see Fig. 10u). This exposes the contact pads of the chips 18, which are then accessible for further interconnection. This interconnection, which is required in order to have the signals and power transferred among the chips 18, may be achieved by wires, in the example given formed by metal tracks 43, which can be straight or curved, and can be long tracks with connections at each electrode of each chip 18 or shorter tracks connecting electrodes of not all chips, e.g. two adjacent chips 18. The metal may be Pt, which may then be applied by plating or lift off. For forming the metal tracks 43, first a photoresist may be deposited and patterned as illustrated in Fig. 10v. Then the metal layer to form the metal tracks 43 may be deposited and the patterned photoresist layer 44 may be removed (see Fig. 10w).

Still aiming at improving the strength of the metal interconnections or tracks 43, it is possible to add an additional material layer 45, such that a sort of a sandwich configuration is formed wherein the metal tracks 43 are embedded between two layers 41, 45 of material. This is illustrated in Fig. 10x.

Finally an additional layer of biocompatible material 46 may be applied over the structure to cover the complete structure (see Fig. 10y). This layer of biocompatible material 46 may be patterned according to the desired final shape of the structure.

In a next step, the carrier wafer 16 may finally be released by removing the sacrificial layer 30 (see Fig. 10z). This may be done with any suitable method such as anodic dissolution, wet etch, dry etch,....

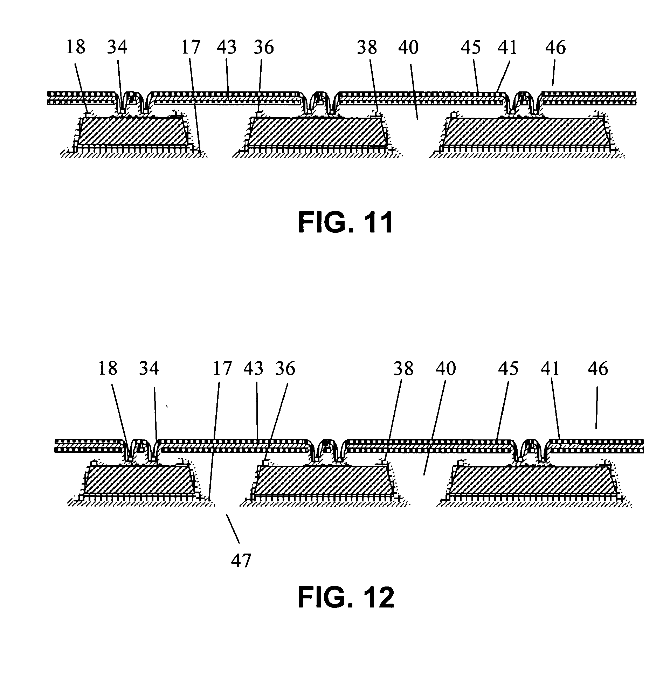

The consequence of the release of the carrier wafer 16 is that a free standing microelectronic device 20 is released (see Fig. 11). This microelectronic device 20 comprises chips 18 embedded in a insulating material and a metal. The metal encapsulation brings the electrical signal from the top contact pads of the chips 18 on one side of the microelectronic device 20 to the back electrodes 17 which can act as an electrode, stimulator and/or sensor on the other opposite side of the microelectronic device.

It has to be noted that according to embodiments of the invention and as can be seen from Fig. 10z, the back electrode 17 is part of the encapsulation of the chips 18. The encapsulation may also be referred to as hermetic package or package. The packaged microelectronic device may have a thickness of lower than 200 µm, lower than 100 µm, lower than 50 µm, lower than 20 µm or lower than 10 µm.

A possible further processing may be over moulding in a preformed mould, enabling the possibility to bring the structure in a predefined shape. This overmoulding may be provided with openings for the electrode contacts. A microelectronic device 20 comprising overmoulding structures 47 is illustrated in Fig. 12.

Furthermore, according to embodiments of the invention, a biocompatible layer may be provided on top of the back electrode 10.

It is to be understood that although preferred embodiments, specific constructions and configurations, as well as materials, have been discussed herein for devices according to the present invention, various changes or modifications in form and detail may be made without departing from the scope of this invention as defined by the appended claims. For example, the microelectronic device 20 has been described by means of a microelectronic device 20 suitable for being implanted in a human body. It has to be understood that the microelectronic device 20 may also be any other microelectronic device different from devices to be implanted for which, for example, costs and complexity of the device have to be kept low. In this case, less care has to be taken for the materials used to be biocompatible and hence, the method for manufacturing such microelectronic devices 20 may have more freedom with respect to choice of materials.

## Claims

1. A packaged microelectronic device (20) comprising at least one electrode (10) comprising a chip (18) embedded in a package, the chip (18) comprising:
- a back electrode (17) located at a first side of the chip (18), and
- electronic circuitry (14) located at a second side of the chip (18), the second side being opposite to the first side,
wherein the back electrode (17) is part of the package.

2. A packaged microelectronic device (20) according to claim 1, the packaged microelectronic device (20) comprising a first number of electrodes (10), wherein the microelectronic device (20) furthermore comprises a second number of wires (12) for connecting the electrodes (10) to a drive circuit and to neighbouring electrodes (10), wherein the first number is higher than the second number.

3. A packaged microelectronic device (20) according to claim 2, wherein the wires (12) are formed by conductive tracks (43).

4. A packaged microelectronic device (20) according to claim 3, wherein the conductive tracks (43) are formed by a stretchable interconnect (24), the stretchable interconnect comprising a horse-shoe shaped interconnection (25) embedded in a silicone carrier (26).

5. A packaged microelectronic device (20) according to any of the previous claims, wherein the at least one electrode (10) is embedded in a biocompatible layer (40).

6. A packaged microelectronic device (20) according to claim 5, wherein the biocompatible layer comprises silicone.

7. A packaged microelectronic device (20) according to any of the previous claims, wherein the packaged microelectronic device (20) furthermore comprises at least one conductive interconnect (38) between the electronic circuitry (14) and the back electrode (17).

8. A packaged microelectronic device (20) according to any of the previous claims, furthermore comprising a biocompatible layer on top of the at least one back electrode (17).

9. A packaged microelectronic device (20) according to any of the previous claims, wherein the at least one back electrode (17) is adapted for performing the function of sensing and/or stimulation.

10. A packaged microelectronic device (20) according to any of the previous claims, wherein the electronic circuitry (14) is adapted for providing sensing and/or stimulation signals from/towards the back electrode (17).

11. Use of the packaged microelectronic device (20) according to any of the previous claims in medical or biomedical implants.

12. A method for forming a packaged microelectronic device (20), the method comprising
- providing a first carrier wafer (16) comprising at least one back electrode (17),
- providing a wafer (13) comprising electronic circuitry (14) comprising at least one chip (18),
- attaching the first carrier wafer (16) to the wafer (13) to form a structure, such that the at least one back electrode (17) is located at a first side of the structure and the electronic circuitry (14) is located at a second side, opposite to the first side, of the structure,
- separating the chips (18) from each other such that at least one electrode (10) is formed, the electrode (10) comprising a chip (18) and a back electrode (17), and
- removing the first carrier wafer (16) so as to release the at least one back electrode (17).

13. A method according to claim 12, wherein providing a wafer (13) comprising electronic circuitry (14) comprises:
- attaching a substrate comprising electronic circuitry (14) to a second carrier wafer (15), and
- thinning the substrate so as to form the wafer (13) comprising the electronic circuitry (14).

14. A method accordion to claim 12 or 13, wherein providing a first carrier wafer (16) comprising at least one back electrode (17) is performed by:
- providing a carrier wafer (16),
- onto the carrier wafer (16) depositing a conductive layer, and
- patterning the conductive layer so as to form the at least one back electrode (17).

15. A method according to any of claims 12 to 14, the method furthermore comprising:
- providing conductive interconnects (38) between the electronic circuitry (14) and the back electrode (17).

16. A method according to any of claims 12 to 15, the packaged microelectronic device (20) comprising a plurality of electrodes (10), the method furthermore comprising:
- providing wires (12) for interconnecting the electrodes (10) and for connecting them to a driving circuit (11).

17. A method according to claim 16, wherein providing wires (12) is performed by providing conductive tracks (43).

18. A method according to claim 17, wherein providing conductive tracks (43) is performed by providing stretchable interconnects (24), the stretchable interconnects (24) comprising a horse-shoe shaped interconnection (25) embedded in a silicone carrier (26).

19. A method according to any of claims 12 to 18, wherein the method furthermore comprises providing a biocompatible layer (40) for embedding the at least one electrode (10).

20. A method according to any claims 12 to 19, wherein the at least one back electrode (17) is adapted for performing the function of sensing and/or stimulation.

21. A method according to any claims 12 to 20, furthermore comprising providing a biocompatible layer on top the at least one back electrode (17).

22. A method according to any of claims 12 to 21, wherein removing the first carrier wafer (16) is performed by removing a sacrificial layer (30) present in between the first carrier wafer (16) and the at least one back electrode(17).

23. A method according to any of claims 12 to 22, wherein the electronic circuitry (14) is adapted for providing sensing and/or stimulation signals from/towards the back electrode (17).
